# EUROPEAN PATENT APPLICATION

(11) **EP 2 123 629 A1**
(43) Date of publication of application: **25.11.2009**
(21) Application number: 08711159.7
(22) Date of filing: 13.02.2008
(51) Int. Cl.: C07C 227/34, C07C 229/22, C07B 57/00

(54) **METHOD FOR PURIFYING 4-HYDROXYISOLEUCINE**

(30) Priority: 22.02.2007 JP 2007042711; 31.05.2007 JP 2007144578
(71) Applicant: Ajinomoto Co., Inc., Tokyo 104-8315 (JP)
(72) Inventor: TOKUMASU, Munetaka, Kawasaki-shi Kanagawa 210-8681 (JP)
(74) Representative: Nicholls, Kathryn Margaret
(86) International application number: PCT/JP2008/052301
(87) International publication number: WO 2008/102671

(57) **Abstract**

The present invention aims to provide a method of conveniently isolating and purifying as well as separating and removing (2S,3R,4S)-4-hydroxyisoleucine at a high purity and in a high yield. Specifically, the present invention discloses a purification method of (2S,3R,4S)-4-hydroxyisoleucine or a chemically acceptable salt thereof, which includes the following steps (a), (b) and (c):
(a) a step of reacting (2S,3R,4S)-4-hydroxyisoleucine or a chemically acceptable salt thereof in a mixture with an aldehyde compound represented by the formula: Q-CHO wherein Q is an aryl group having a carbon number of 6 to 14, an alkyl group having a carbon number of 1 to 10, a cycloalkyl group having a carbon number of 3 to 10 or a 5- to 10-membered heterocyclic group, each of which optionally has substituent(s), or an equivalent form thereof to give a compound represented by the formula (1) 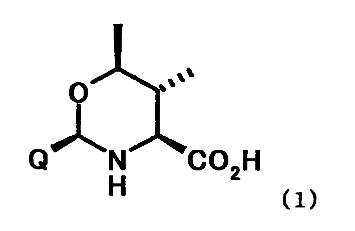 wherein Q is as defined above,
(b) a step of extracting the compound represented by the formula (1) with an organic solvent, and
(c) a step of converting the compound represented by the formula (1) to (2S,3R,4S)-4-hydroxyisoleucine or a chemically acceptable salt thereof.

## Description

### Technical Field

The present invention relates to a method of isolating and purifying 4-hydroxyisoleucine. More particularly, it relates to a method of isolating and purifying as well as separating and removing (2S,3R,4S)-4-hydroxyisoleucine, which utilizes conversion to an aminal form.

### Background Art

4-Hydroxyisoleucine is one of the nonprotein amino acids, and known to be extracted from the seeds of Fenugreek (Trigonella foenum-graecum L.) and the like (non-patent documents 1, 2 and 3).
With regard to this 4-hydroxyisoleucine, blood glucose level-dependent insulin secretion-promoting activity (patent document 1, non-patent document 4), insulin sensitivity-enhancing action (patent documents 2, 3, non-patent document 5), glycogen formation-promoting activity in muscle (patent document 4, non-patent document 6), skin and scalp-protecting action (patent document 5), fat absorption-suppressive action (patent document 6), antiobesity action (patent document 7) and the like have been reported, and 4-hydroxyisoleucine is useful for pharmaceutical products, health food materials, cosmetic materials and the like, which utilize these actions. Furthermore, it is useful as a synthetic intermediate for various pharmaceutical products.
At present, as a method for obtaining 4-hydroxyisoleucine, extraction from the seeds of Fenugreek (patent documents 8 and 9, non-patent documents 1, 2 and 3) and chemical synthesis (patent documents 10, 11, non-patent document 7) are known.
The extraction method requires a complicated operation such as chromatography using silica gel and ion exchange resin, recrystallization and the like for separation from saponins and other amino acids. Particularly, 4-hydroxyisoleucine contained in the seeds of Fenugreek contains, besides (2S,3R,4S)-4-hydroxyisoleucine considered to be an important stereoisomer for exerting the aforementioned action, (2R,3R,4S)-4-hydroxyisoleucine, which is a different stereoisomer, at a ratio of about 9:1 (non-patent document 2), and separation of such stereoisomer is not easy even when chromatography and recrystallization are used repeatedly.
In patent document 8, 0.6 g of (2S,3R,4S)-4-hydroxyisoleucine having a purity of 99% is obtained from defatted seeds of Fenugreek (100 g) by chromatography using an ion exchange resin and recrystallization; however, this recovery rate is considered to be not high. Particularly, due to the property, recrystallization always causes loss of the object product into the filtrate, and the recovery rate keeps decreasing every time it is performed.
Also in synthetic methods, a stereoisomer is often contained due to side reaction and the like, and such impurity requires a complicated operation such as HPLC and recrystallization for removal. Such operation is associated with difficulty in large-scale synthesis from the aspects of cost and facility, as well as a decrease in the yield.
Thus, conventional purification methods of 4-hydroxyisoleucine have problems, and an efficient and convenient method has been desired.
patent document 1: EP Patent No. 587476
patent document 2: WO2001/015689
patent document 3: WO2005/039626
patent document 4: WO2005/021596
patent document 5: WO2004/087091
patent document 6: JP-A-2006-89449
patent document 7: US Patent application No. 2006/0223884
patent document 8: US Patent No. 5470879
patent document 9: WO2005/084323
patent document 10: WO2004/099120
patent document 11: WO2006/051000
non-patent document 1: Phytochemistry, vol. 12, 1707-1711, 1973
non-patent document 2: Phytochemistry, vol. 15, 325, 1976
non-patent document 3: Phytochemistry, vol. 28, 1835-1841, 1989
non-patent document 4: Diabetes, Vol. 47, Issue 2 206-210, 1998
non-patent document 5: American Journal of Physiology Endocrinology and Metabolism, Vol. 287, E463-E471, 2004
non-patent document 6: Amino Acids, vol. 28, 71-76, 2005
non-patent document 7: European Journal of Organic Chemistry, vol.5, 834-839, 2002

### Disclosure of the Invention

### Problems to be Solved by the Invention

The present invention aims to provide a method of isolating and purifying (2S,3R,4S)-4-hydroxyisoleucine or a chemically acceptable salt thereof (hereinafter to be sometimes referred to as "(2S,3R,4S)-HIL") conveniently at a high purity and in a high yield.
In addition, the present invention aims to provide a method of separating and removing (2S,3R,4S)-4-hydroxyisoleucine conveniently from a mixture. Means of Solving the Problems

The present inventors have found that an aminal form of (2S,3R,4S)-4-hydroxyisoleucine can be separated conveniently and finely from a stereoisomer thereof or other compounds such as saponins derived from crude extracts, other amino acids and the like by converting (2S,3R,4S)-4-hydroxyisoleucine into an aminal form and extracting the same with an organic solvent, which resulted in the completion of the present invention.
Accordingly, the present invention is as follows.
[1] A method of purifying (2S,3R,4S)-4-hydroxyisoleucine or a chemically acceptable salt thereof, which comprises the following steps (a), (b) and (c):
   (a) a step of reacting (2S,3R,4S)-4-hydroxyisoleucine or a chemically acceptable salt thereof in a mixture with an aldehyde compound represented by the formula: Q-CHO wherein Q is an aryl group having a carbon number of 6 to 14, an alkyl group having a carbon number of 1 to 10, a cycloalkyl group having a carbon number of 3 to 10 or a 5- to 10-membered heterocyclic group, each of which optionally has substituent(s), or an equivalent form thereof to give a compound represented by the formula (1)

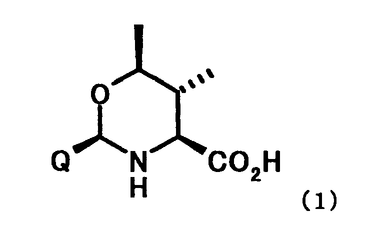

wherein Q is as defined above,
(b) a step of extracting the compound represented by the formula (1) with an organic solvent, and
(c) a step of converting the compound represented by the formula (1) to (2S,3R,4S)-4-hydroxyisoleucine or a chemically acceptable salt thereof.

[2] The method of the above-mentioned [1], wherein an aldehyde compound represented by the formula: Q-CHO is used for the reaction of step (a).
[3] The method of the above-mentioned [1], comprising removing impurities from the compound represented by the formula (1) by the extraction step of step (b).
[4] The method of the above-mentioned [1] or [2], wherein Q is a phenyl group, a cyclohexyl group, a naphthyl group, a pyridyl group, a thienyl group, a furyl group or a pyrrolyl group, each of which optionally has substituent(s).
[5] The method of the above-mentioned [4], wherein Q in the formula (1) is a phenyl group optionally having substituent(s).
[6] The method of the above-mentioned [4] or [5], wherein the substituent optionally possessed by Q is selected from the group consisting of
   (i) an alkyl group having a carbon number of 1 to 6,
   (ii) an alkoxy group having a carbon number of 1 to 6,
   (iii) an alkanoyl group having a carbon number of 2 to 7,
   (iv) an aryl group having a carbon number of 6 to 10,
   (v) an aryloxy group having a carbon number of 6 to 10,
   (vi) a halogeno group,
   (vii) a halogenoalkyl group having a carbon number of 1 to 6,
   (viii) a nitro group,
   (ix) a formyl group, and
   (x) a cyano group,
   provided that the above-mentioned (i) and (vii) are not substituents of an alkyl group having a carbon number of 1 to 10.
[7] The method of the above-mentioned [1], wherein the organic solvent of step (b) is at least one kind of alkyl acetate.
[8] The method of the above-mentioned [1], which uses, in step (c), at least one kind of nucleophilic reagent selected from the group consisting of water, hydroxylamine optionally substituted by alkyl having a carbon number of 1 to 4, ammonia, alkylamine having a carbon number of 1 to 4, dialkylamine having a carbon number of 2 to 8, hydrazine optionally substituted by substituent(s) selected from an alkyl group having a carbon number of 1 to 4, an aryl group having a carbon number of 6 to 10 and an alkanoyl group having a carbon number of 2 to 7, quaternary ammonium halide substituted by alkyl having a carbon number of 1 to 6, and a chemically acceptable salt thereof.
[9] The method of the above-mentioned [1], wherein the conversion to (2S,3R,4S)-4-hydroxyisoleucine or a chemically acceptable salt thereof is performed in at least one kind of alkyl acetate in step (c).
[10] The method of the above-mentioned [9], wherein the at least one kind of alkyl acetate is ethyl acetate.
[11] A method of separating and removing (2S,3R,4S)-4-hydroxyisoleucine or a chemically acceptable salt thereof, comprising the following steps (a) and (b):
   (a) a step of reacting (2S,3R,4S)-4-hydroxyisoleucine or a chemically acceptable salt thereof in a mixture with an aldehyde compound represented by the formula: Q-CHO wherein Q is an aryl group having a carbon number of 6 to 14, an alkyl group having a carbon number of 1 to 10, a cycloalkyl group having a carbon number of 3 to 10 or a 5- to 10-membered heterocyclic group, each of which optionally has substituent(s), or an equivalent form thereof to give a compound represented by the formula (1)

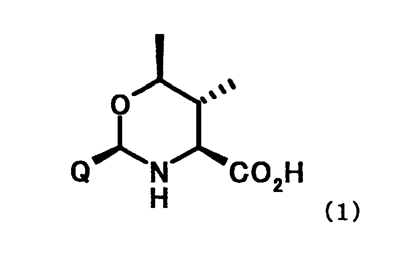

wherein Q is as defined above, and
(b) a step of extracting the compound represented by the formula (1) with an organic solvent.

[12] The method of the above-mentioned [11], wherein an aldehyde compound represented by the formula: Q-CHO is used for the reaction in step (a).
[13] The method of the above-mentioned [11] or [12], wherein Q in the formula (1) is a phenyl group, a cyclohexyl group, a naphthyl group, a pyridyl group, a thienyl group, a furyl group or a pyrrolyl group, each of which optionally has substituent(s).
[14] The method of the above-mentioned [13], wherein Q in the formula (1) is a phenyl group optionally having substituent(s).
[15] The method of the above-mentioned [13] or [14], wherein the substituent optionally possessed by Q is selected from the group consisting of
   (i) an alkyl group having a carbon number of 1 to 6,
   (ii) an alkoxy group having a carbon number of 1 to 6,
   (iii) an alkanoyl group having a carbon number of 2 to 7,
   (iv) an aryl group having a carbon number of 6 to 10,
   (v) an aryloxy group having a carbon number of 6 to 10,
   (vi) a halogeno group,
   (vii) a halogenoalkyl group having a carbon number of 1 to 6,
   (viii) a nitro group,
   (ix) a formyl group, and
   (x) a cyano group,
   provided that the above-mentioned (i) and (vii) are not used as substituents of an alkyl group having a carbon number of 1 to 10.
[16] The method of the above-mentioned [11], wherein the organic solvent in step (b) is at least one kind of alkyl acetate.

### Effect of the Invention

The method of the present invention is useful as a method of isolating and purifying (2S,3R,4S)-4-hydroxyisoleucine or a chemically acceptable salt thereof, and can provide (2S,3R,4S)-HIL having a high purity in a high yield more efficiently than chromatography and recrystallization.
More specifically, (2S,3R,4S)-HIL can be isolated efficiently from a mixture containing 4-hydroxyisoleucine, which is derived from a Fenugreek extract, a fermentation broth obtained by culturing microorganism and the like. In addition, (2S,3R,4S)-HIL can be isolated efficiently from a mixture containing 4-hydroxyisoleucine, which is obtained by chemical synthesis.
Moreover, the method of the present invention is also useful as a method of separating and removing (2S,3R,4S)-HIL, and can efficiently remove (2S,3R,4S)-HIL from a mixture of compounds. As a result, the method can increase the purity of other compounds in a mixture, for example, a stereoisomer thereof, i.e., (2R,3R,4S)-4-hydroxyisoleucine, or a chemically acceptable salt thereof (hereinafter sometomes to be also referred to as "(2R,3R,4S)-HIL").

### Best Mode for Carrying out the Invention

The present invention is explained in detail in the following.
In the method of the present invention, (2S,3R,4S)-4-hydroxyisoleucine may be in the form of a chemically acceptable salt. The "chemically acceptable salt" is not particularly limited as long as it does not adversely influence the method of the present invention. Examples thereof include salts with ammonia; alkali metal such as sodium, potassium and the like; alkaline earth metal such as calcium, magnesium and the like; aluminum; zinc; organic amine such as triethylamine, ethanolamine, morpholine, pyrrolidine, piperidine, piperazine, dicyclohexylamine and the like; basic amino acid such as arginine, lysine and the like; inorganic acid such as hydrochloric acid, sulfuric acid, phosphoric acid, nitric acid, hydrobromic acid and the like; organic carboxylic acid such as acetic acid, citric acid, benzoic acid, maleic acid, fumaric acid, tartaric acid, succinic acid, tannic acid, butyric acid, hibenzoic acid, pamoic acid, enanthic acid, decanoic acid, teoclic acid, salicylic acid, lactic acid, oxalic acid, mandelic acid, malic acid and the like; organic sulfonic acid such as methanesulfonic acid, benzenesulfonic acid, p-toluenesulfonic acid, etc. and the like.

The mixture to be the target of the purification method or separation method of the present invention may be any as long as it is a chemical substance mixture containing (2S,3R,4S)-HIL, and the content ratio thereof and the composition of other substances are subject to no limitation. Examples of the mixture to be the target of the method of the present invention include a plant tissue extract, an animal tissue extract, fermentation broth obtained by culturing a microorganism, a mixture derived from a reaction mixture after chemical synthesis and the like.
In addition, the purification method of the present invention can also be applied to remove a small amount of impurity in a comparatively pure (2S,3R,4S)-HIL crude product.
In the purification method of the present invention, the purity of (2S,3R,4S)-HIL obtained thereby only needs to be improved from before the purification and is not particularly limited. In a preferable embodiment of the present method, a substantially pure (2S,3R,4S)-HIL, for example, that is free of an impurity peak when measured by ¹H-NMR (300 M Hz) is provided.

The compound represented by the formula (1) of the present invention is an aminal form of (2S,3R,4S)-HIL, wherein, in the formula (1), Q is an aryl group having a carbon number of 6 to 14, an alkyl group having a carbon number of 1 to 10, a cycloalkyl group having a carbon number of 3 to 10 or a 5-to 10-membered heterocyclic group, each optionally having substituent(s).
Examples of the "aryl group having a carbon number of 6 to 14" of the above-mentioned "aryl group having a carbon number of 6 to 14, which optionally has substituent(s)" include a phenyl group, a naphthyl group, an anthracenyl group, an phenanthrenyl group, a biphenyl group and the like. Preferred is an aryl group having a carbon number of 6 to 10 (e.g., phenyl group, naphthyl group, biphenyl group etc.), and more preferred is a phenyl group.
Examples of the "alkyl group having a carbon number of 1 to 10" of the above-mentioned "alkyl group having a carbon number of 1 to 10, which optionally has substituent(s)" include a straight chain or branched chain alkyl group having a carbon number of 1 to 10, preferably a straight chain or branched chain alkyl group having a carbon number of 1 to 6 (e.g., methyl group, ethyl group, n-propyl group, isopropyl group, n-butyl group, sec-butyl group, isobutyl group, tert-butyl group, pentyl group, hexyl group etc.).
Examples of the "cycloalkyl group having a carbon number of 3 to 10" of the above-mentioned "cycloalkyl group having a carbon number of 3 to 10, which optionally has substituent(s)" include a cyclopropyl group, a cyclobutyl group, a cyclopentyl group, a cyclohexyl group, a cycloheptyl group, a cyclooctyl group, a cyclononyl group, a cyclodecyl group and the like. Preferred is a cycloalkyl group having a carbon number of 5 to 8 (e.g., cyclopentyl group, cyclohexyl group, cycloheptyl group, cyclooctyl group etc.), and more preferred is a cyclohexyl group.

Examples of the "5- to 10-membered heterocyclic group" of the above-mentioned "5- to 10-membered heterocyclic group, which optionally has substituent(s)" include a 5- to 10-membered aromatic heterocyclic group or non-aromatic heterocycle containing, besides carbon atom, 1 to 4 hetero atoms selected from an oxygen atom, a sulfur atom and a nitrogen atom, and a fused ring group thereof.
Examples of the aromatic heterocyclic group or a fused ring group thereof include 2- or 3-thienyl, 2- or 3-furyl, 1-, 2- or 3-pyrrolyl, 1-, 2-, 4- or 5-imidazolyl, 2-, 4- or 5-oxazolyl, 2-, 4- or 5-thiazolyl, 1-, 3-, 4- or 5-pyrazolyl, 3-, 4- or 5-isoxazolyl, 3-, 4- or 5-isothiazolyl, 1,2,4-triazol-1-, 3-, 4- or 5-yl, 1,2,3-triazol-1-, 2- or 4-yl, 1H-tetrazol-1-or 5-yl, 2H-tetrazol-2- or 5-yl, 2-, 3- or 4-pyridyl, 2-, 4-or 5-pyrimidinyl, 1-, 2-, 3-, 4-, 5-, 6- or 7-indolyl, 2-, 3-, 4-, 5-, 6- or 7-benzofuryl, 2-, 3-, 4-, 5-, 6- or 7-benzothienyl, 1-, 2-, 4-, 5-, 6- or 7-benzimidazolyl, 2-, 3-, 4-, 5-, 6-, 7- or 8-quinolyl, 1-, 3-, 4-, 5-, 6-, 7- or 8-isoquinolyl and the like.
Examples of the nonaromatic heterocyclic group or a fused ring group thereof include 2- or 3-dihydrothienyl, 2- or 3-dihydrofuryl, 1-, 2- or 3-pyrrolinyl, 1-, 2-, 4- or 5-imidazolinyl, 2-, 4- or 5-oxazolinyl, 2-, 4- or 5-thiazolinyl, 1-, 3-, 4- or 5-pyrazolinyl, 3-, 4- or 5-isoxazolinyl, 3-, 4-or 5-isothiazolinyl, 1,2,4-triazolin-1-, 3-, 4- or 5-yl, 1,2,3-triazolin-1-, 2- or 4-yl, 1H-tetrazolin-1- or 5-yl, 2H-tetrazolin-2- or 5-yl, 2-, 3- or 4-dihydropyridyl, 2-, 4- or 5-dihydropyrimidinyl, 1-, 2-, 3-, 4-, 5-, 6- or 7-dihydroindolyl, 2-, 3-, 4-, 5-, 6- or 7-dihydrobenzofuryl, 2-, 3-, 4-, 5-, 6- or 7-dihydrobenzothienyl, 1-, 2-, 4-, 5-, 6- or 7-dihydrobenzimidazolyl, 2-, 3-, 4-, 5-, 6-, 7- or 8-dihydroquinolyl, 1-, 3-, 4-, 5-, 6-, 7- or 8-dihydroisoquinolyl, 2- or 3-tetrahydrothienyl, 2- or 3-tetrahydrofuryl, 1-, 2- or 3-tetrahydropyranyl, 1-, 2- or 3-pyrrolidinyl, 1-, 2-, 4- or 5-imidazolidinyl, 2-, 4- or 5-oxazolidinyl, 2-, 4- or 5-thiazolidinyl, 1-, 3-, 4- or 5-pyrazolidinyl, 3-, 4- or 5-isoxazolidinyl, 3-, 4- or 5-isothiazolidinyl, 1,2,4-triazolidin-1-, 3-, 4- or 5-yl, 1,2,3-triazolidin-1-, 2- or 4-yl, 1H-tetrazolidin-1- or 5-yl, 2H-tetrazolidin-2- or 5-yl, 2-, 3- or 4-piperidinyl, 1- or 2-piperazinyl, 1-, 2- or 3-morpholinyl, 1-, 2- or 3-thiomorpholinyl, 2-, 3-, 4-, 5-, 6-, 7- or 8-tetrahydroquinolyl, 1-, 3-, 4-, 5-, 6-, 7- or 8-tetrahydroisoquinolyl and the like.
Preferable examples of the "heterocyclic group" include a thienyl group, a furyl group, an oxazolyl group, a thiazolyl group, an isoxazolyl group, an isothiazolyl group, a pyridyl group, an indolyl group, a benzofuryl group, a benzothienyl group, a dihydrothienyl group, a dihydrofuryl group, a tetrahydrothienyl group, a tetrahydrofuryl group, a tetrahydropyranyl group and the like, with more preference given to a thienyl group, a furyl group, a pyridyl group and the like.

In another embodiment, Q is an aryl group having a carbon number of 6 to 14, an alkyl group having a carbon number of 1 to 10 or a 5- to 10-membered heterocyclic group, each of which optionally has substituent(s).

Examples of the substituent of the above-mentioned "aryl group having a carbon number of 6 to 14, alkyl group having a carbon number of 1 to 10, cycloalkyl group having a carbon number of 3 to 10 or 5- to 10-membered heterocyclic group, each of which optionally has substituent(s)" for Q include:
(i) an alkyl group having a carbon number of 1 to 6,
(ii) an alkoxy group having a carbon number of 1 to 6,
(iii) an alkanoyl group having a carbon number of 2 to 7,
(iv) an aryl group having a carbon number of 6 to 10,
(v) an aryloxy group having a carbon number of 6 to 10,
(vi) a halogeno group,
(vii) a halogenoalkyl group having a carbon number of 1 to 6,
(viii) a nitro group,
(ix) a formyl group, and
(x) a cyano group,
provided that the above-mentioned (i) and (vii) are not substituents of an alkyl group having a carbon number of 1 to 10.

In another embodiment, the above-mentioned substituent is selected from
(i) an alkyl group having a carbon number of 1 to 6,
(ii) an alkoxy group having a carbon number of 1 to 6,
(iii) an alkanoyl group having a carbon number of 2 to 7,
(iv) an aryl group having a carbon number of 6 to 10,
(v) an aryloxy group having a carbon number of 6 to 10,
(vi) a halogeno group,
(vii) a halogenoalkyl group having a carbon number of 1 to 6,
(viii) a nitro group, and
(ix) a formyl group,
provided that the above-mentioned (i) and (vii) are not substituents of an alkyl group having a carbon number of 1 to 10.

The above-mentioned "(i) alkyl group having a carbon number of 1 to 6" is a straight chain or branched chain alkyl group having a carbon number of 1 to 6, and includes, for example, a methyl group, an ethyl group, an n-propyl group, an isopropyl group, an n-butyl group, a sec-butyl group, an isobutyl group, a tert-butyl group, a pentyl group, a hexyl group and the like, with preference given to a methyl group, an ethyl group, an n-propyl group and an isopropyl group.
The above-mentioned "(ii) alkoxy group having a carbon number of 1 to 6" is a group wherein the above-mentioned "alkyl group having a carbon number of 1 to 6" is bonded to an oxygen atom, and includes, for example, a methoxy group, an ethoxy group, an n-propoxy group, an isopropoxy group, an n-butoxy group, a sec-butoxy group, an isobutoxy group, a tert-butoxy group, a pentyloxy group, a hexyloxy group and the like, with preference given to a methoxy group, an ethoxy group, an n-propoxy group and an isopropoxy group.
The above-mentioned "(iii) alkanoyl group having a carbon number of 2 to 7" is a group wherein the above-mentioned "alkyl group having a carbon number of 1 to 6" is bonded to a carbonyl group, and includes, for example, an acetyl group, an ethylcarbonyl group, an n-propylcarbonyl group, an isopropylcarbonyl group, an n-butylcarbonyl group, a sec-butylcarbonyl group, an isobutylcarbonyl group, a tert-butylcarbonyl group, a pentylcarbonyl group, a hexylcarbonyl group and the like, with preference given to an acetyl group, an ethylcarbonyl group, an n-propylcarbonyl group and an isopropylcarbonyl group.

Examples of the above-mentioned "(iv) aryl group having a carbon number of 6 to 10" include a phenyl group, a naphthyl group, a biphenyl group and the like, with preference given to a phenyl group.
The above-mentioned "(v) aryloxy group having a carbon number of 6 to 10" is a group wherein the above-mentioned "aryl group having a carbon number of 6 to 10" is bonded to an oxygen atom, and includes, for example, a phenoxy group, a naphthyloxy group, a biphenyloxy group and the like, with preference given to a phenoxy group.

Examples of the above-mentioned "(vi) halogeno group " include a fluorine atom, a chlorine atom, a bromine atom, an iodine atom and the like, with preference given to a fluorine atom, a chlorine atom and a bromine atom.
The above-mentioned "(vii) halogenoalkyl group having a carbon number of 1 to 6" is a group wherein the above-mentioned "alkyl group having a carbon number of 1 to 6" is substituted by a substitutable number, preferably 1 - 5, of one or more kinds of the above-mentioned "halogeno groups", and includes, for example, a fluoromethyl group, a difluoromethyl group, a trifluoromethyl group, a monochloromethyl group, a dichloromethyl group, a trichloromethyl group, a chlorofluoromethyl group, a 1-fluoroethyl group, a 1,1-difluoroethyl group, a 1,1,1-trifluoroethyl group, a pentafluoroethyl group, a pentachloroethyl group and the like, with preference given to a trifluoromethyl group and a trichloromethyl group.
When the above-mentioned "aryl group having a carbon number of 6 to 14, alkyl group having a carbon number of 1 to 10, cycloalkyl group having a carbon number of 3 to 10 or 5-to 10-membered heterocyclic group" has substituent(s) selected from the above-mentioned substituents (i) - (x), the position and the number of the substituent are not limited unless otherwise specified. When the number of the above-mentioned substituents (i) - (x) is two or more, the substituents may be the same or different. However, the above-mentioned (i) and (vii) are not substituents of the "alkyl group having a carbon number of 1 to 10".
Moreover, when the above-mentioned "aryl group having a carbon number of 6 to 14, alkyl group having a carbon number of 1 to 10 or 5- to 10-membered heterocyclic group" has substituent(s) selected from the above-mentioned substituents (i) - (ix), the position and the number of the substituent are not limited unless otherwise specified. When the number of the above-mentioned substituents (i) - (ix) is two or more, the substituents may be the same or different. However, the above-mentioned (i) and (vii) are not substituents of the "alkyl group having a carbon number of 1 to 10"

As Q, a group having a certain level of bulkiness and liposolubility is preferable. More specifically, a phenyl group, a cyclohexyl group, a naphthyl group, a pyridyl group, a thienyl group, a furyl group and a pyrrolyl group, each optionally having substituent(s), are preferable, with more preference given to a phenyl group optionally having substituent(s). When these groups have substituent(s), examples of the "substituent" include substituents selected from the above-mentioned (i) - (x) and the like.
In another embodiment, Q is preferably a phenyl group, a naphthyl group, a pyridyl group, a thienyl group, a furyl group or a pyrrolyl group, each optionally having substituent (s).
Q is particularly preferably a nitrophenyl group, a phenyl group or a cyclohexyl group.
In another embodiment, Q is particularly preferably a nitrophenyl group or a phenyl group.

Now the method of the present invention is explained in more detail.
The method of the present invention has been completed based on the finding that (2S,3R,4S)-HIL is finely converted to a compound represented by the formula (1), i.e., an aminal form (1) of (2S,3R,4S)-HIL (hereinafter sometimes to be simply referred to as "a compound represented by the formula (1)" or "aminal form (1)") by an aminalization reaction, and the compound of the formula (1) is extracted well in an organic solvent.
For example, when an aldehyde compound or an equivalent thereto is added to a solution containing (2S,3R,4S)-HIL, aminal form (1) of (2S,3R,4S)-HIL is produced. The (2S,3R,4S)-HIL by itself is difficult for extraction with an organic solvent. However, the resulting aminal form (1) has increased liposolubility and can be extracted with an organic solvent easily. As a result, extraction and purification by partitioning of aminal form (1) become possible.
On the other hand, (2R,3R,4S)-HIL, which is a stereoisomer of (2S,3R,4S)-HIL, hardly forms the corresponding aminal form in the above-mentioned reaction. That is, when aminalization is performed using a mixture of (2S,3R,4S)-HIL and (2R,3R,4S)-HIL under general conditions, (2S,3R,4S)-HIL alone is selectively aminalized. Therefore, the purification method of the present invention enables even the removal of (2R,3R,4S)-HIL, which is a compound structurally extremely similar to (2S,3R,4S)-HIL.

Each step of the method of the present invention is explained in detail in the following.

### step (a)

In the method of the present invention, firstly, a mixture containing (2S,3R,4S)-HIL is subjected to an aminalization reaction in step (a).
The aminalization reaction is performed by reacting (2S,3R,4S)-HIL in a mixture with an aldehyde compound represented by the formula Q-CHO (Q is as defined above) or an equivalent thereto. In this case, an aldehyde compound itself is preferably used rather than an equivalent thereto.
Here, the equivalent to the aldehyde compound is a synthesis equivalent that acts like the aldehyde compound Q-CHO (Q is as defined above) in this aminalization reaction. Such equivalent to the aldehyde compound includes an acetal form, a hemiacetal form, an aminal, a dihalogenomethyl form and the like corresponding to a compound represented by the formula Q-CHO (Q is as defined above), and an acetal form and a hemiacetal form are particularly preferable. More specifically, an acetal or hemiacetal compound represented by the formula Q-CH(OR')OR" [Q is as defined above, R' is a hydrogen atom, a methyl group or an ethyl group, and R" is a methyl group or an ethyl group, or R' and R" may be bonded to form an ethylene group] can be mentioned.
An aldehyde compound represented by the formula Q-CHO or an equivalent thereto is preferably added in an amount of 1 - 5 equivalents relative to the content of 4-hydroxyisoleucine in a mixture. Here, the "content of 4-hydroxyisoleucine in a mixture" means the content of (2S,3R,4S)-HIL in a mixture, and when (2S,3R,4S)-HIL and a stereoisomer thereof ((2S,3R,4S)-HIL) in the mixture, the content is the total thereof.
The above-mentioned reaction may be performed in the presence of a base where necessary. The base may be an inorganic base or an organic base and, for example, an inorganic base such as potassium hydroxide, sodium hydroxide, lithium hydroxide, sodium carbonate, sodium hydrogen carbonate and the like, an organic base such as triethylamine, diisopropylethylamine, morpholine, pyrrolidine, piperidine, piperazine, dicyclohexylamine, ethanolamine, aqueous ammonia and the like, and the like are preferable.
The concentration of the base in the reaction mixture is preferably about 0.1 mol/L - 2 mol/L, and about 1 - 10 equivalents relative to the content of 4-hydroxyisoleucine in the mixture is preferable.
The above-mentioned reaction may be performed in a solvent where necessary. As the reaction solvent, water, acetonitrile, acetone, tetrahydrofuran, methanol, ethanol, isopropanol or 1,4-dioxane, or a mixed solvent thereof at an optional ratio is preferable.
The temperature of the above-mentioned reaction is preferably 0°C - 50°C.

### step (b)

Then, the obtained aminal form (1) is extracted with an appropriate organic solvent in step (b).
Examples of the organic solvent used for extraction of aminal form (1) include solvents that are not immiscible with water such as alkyl acetate, hexane, heptane, diethyl ether, tert-butyl methyl ether, dichloromethane, chloroform and the like and a mixture of two or more kinds thereof, and at least one kind of alkyl acetate is preferable. Examples of the "alkyl acetate" include alkyl ester of acetic acid having a carbon number of 1 to 10, and alkyl ester of acetic acid having a carbon number of 1 to 4 (e.g., methyl acetate, ethyl acetate, n-propyl acetate, isopropyl acetate, n-butyl acetate, sec-butyl acetate, tert-butyl acetate etc.) is more preferable.
In this case, preferably before extraction of aminal form (1) with an organic solvent, water or base is added to the mixture after the reaction as necessary, to give an aqueous alkaline solution, which is washed with an organic solvent. Unreacted aldehyde, an equivalent thereto etc. can be removed by extraction with an organic solvent (e.g., alkyl acetate explained above etc.).
When the aqueous layer after washing is weakly acidified with an acidic solution, dissociation of the carboxyl group of aminal form (1) can be suppressed and can be extracted with a suitable organic solvent. In this case, since unreacted (2R,3R,4S)-HIL remains in an aqueous layer, aminal form (1) of (2S,3R,4S)-HIL alone is extracted in an organic solvent at a high purity, and the both can be efficiently separated.
As an applicable acidic solution, an aqueous low liposoluble acid solution such as hydrochloric acid, sulfuric acid, phosphoric acid, nitric acid, hydrobromic acid, acetic acid, citric acid, maleic acid, fumaric acid, tartaric acid, succinic acid, butyric acid, lactic acid, oxalic acid, malic acid, methane sulfonic acid and the like are preferable.
In the above-mentioned manner, impurities such as unreacted aldehyde, an equivalent thereto, (2R,3R,4S)-HIL and the like can be removed from aminal form (1).
The aminal form (1) extracted with an organic solvent can be further purified by a generally-employed purification method such as crystallization, slurry washing, drying and the like before proceeding to the deaminalization reaction in the next step (c).

### step (c)

Then, the aminal form (1) is subjected to a deaminalization reaction to reconstruct (2S,3R,4S)-HIL in step (c).
The method of deaminalization reaction of aminal form (1) is not particularly limited and, for example, deaminalization proceeds to allow conversion to (2S,3R,4S)-HIL even by only placing aminal form (1) in the water-containing extraction solvent obtained in the above-mentioned step (b).
Preferably, this deaminalization reaction is performed using a nucleophilic reagent.
As applicable nucleophilic reagents, water, hydroxylamine optionally substituted by alkyl group having a carbon number of 1 to 4 (e.g., hydroxylamine), ammonia, alkylamine having a carbon number of 1 to 4 (e.g., methylamine), dialkylamine having a carbon number of 2 to 8 (e.g., dimethylamine), hydrazine optionally substituted by substituent(s) selected from an alkyl group having a carbon number of 1 to 4, an aryl group having a carbon number of 6 to 10 and an alkanoyl group having a carbon number of 2 to 7 (e.g., hydrazine, methylhydrazine, phenylhydrazine, acetylhydrazine), quaternary ammonium halide substituted by alkyl having a carbon number of 1 to 6 (e.g., tetrabutylammonium fluoride, tetrabutylammonium chloride, tetrabutylammonium bromide, tetrabutylammonium iodide) and the like are preferable, and hydroxylamine, methylamine and hydrazine are particularly preferable.
The deaminalization reaction is performed in a solvent as necessary. As the solvent, any solvent can be used as long as it does not adversely influence the reaction, for example, ester solvents (e.g., alkyl acetate similar to those exemplified in the explanation of the above-mentioned step (b)), hydrocarbon solvents (e.g., hexane, heptane), ether solvents (e.g., diethyl ether, tert-butyl methyl ether), halogenated hydrocarbon solvents (e.g., dichloromethane, chloroform), alcoholic solvent (e.g., methanol, ethanol, isopropanol), and a mixed solvent thereof are preferable, alkylester of at least one kind of acetic acid and having a carbon number of 1 to 4 is more preferable, and ethyl acetate is most preferable. (2S,3R,4S)-HIL shows low solubility in ethyl acetate. When the concentration and the like are appropriately set, crystals thereof can be easily obtained as precipitate after the reaction.

Particularly, when Q in the formula (1) is an aryl group having a carbon number of 6 to 14 or a 5- to 10-membered heterocyclic group, each optionally having substituent(s), the deaminalization reaction is preferably also performed by a catalytic reduction.
As a metal catalyst applicable to the catalytic reduction, palladium, rhodium, platinum and the like are preferable.
As a solvent applicable to the catalytic reduction, water, methanol, ethanol or ethyl acetate, and a mixed solvent thereof are preferable.
The thus-reconstructed (2S,3R,4S)-HIL may be further purified by a purification method generally used such as recrystallization, slurry washing, drying and the like.
(2S,3R,4S)-HIL isolated and purified in this manner is useful as a pharmaceutical product (therapeutic agent for diabetes etc.), health food material, cosmetic material and the like, and further useful as a synthetic intermediate for various pharmaceutical products.

For example, when (2S,3R,4S)-HIL is used as an agent for the prophylaxis or treatment of diabetes, it can be administered orally or parenterally (e.g., routes such as intravenous, subcutaneous, intramuscular, suppository, enema, ointment, plaster, sublingual, instillation, inhalation administrations and the like) as it is or as a pharmaceutical composition also containing a pharmaceutically acceptable carrier according to a method known per se. The dose for the above-mentioned object is determined according to the object treatment effect, administration method, treatment duration, age, body weight and the like. For an oral or parenteral route, a general daily dose for an adult by oral administration is 1 µg - 10 g, and 0.01 µg - 1 g by parenteral administration, which are administered in one to plural portions a day. In addition, the content of (2S,3R,4S)-HIL in the above-mentioned pharmaceutical composition is about 0.01 wt% - 100 wt% of the whole composition.

Examples of the pharmaceutically acceptable carrier in the above-mentioned pharmaceutical composition include various organic or inorganic carrier substances conventionally used as a preparation material, such as excipient, lubricant, binder, disintegrant, water-soluble polymer and basic inorganic salt for solid preparations; and solvent, solubilizing agent, suspending agent, isotonicity agent, buffer, soothing agent and the like for liquid preparations. Where necessary, general additives such as preservative, antioxidant, colorant, sweetening agent, souring agent, bubbling agent, flavor and the like can also be used.
Examples of the dosage form of such pharmaceutical composition include tablet, powder, pill, granule, capsule, suppository, liquid, sugar coating agent, depot, syrup, suspension, emulsion, troche, hypoglottis, adhesive preparation, orally disintegrating tablet, inhalant, enteroclysis, ointment, plaster, tape and eye drop, which can be produced according to a conventional method using conventional preparation auxiliary substances.

The above-mentioned pharmaceutical composition can be produced by a method conventionally used in the technical field of preparations, for example, the method described in the Japanese Pharmacopoeia and the like. In the following, specific production methods of preparations are described in detail.
For example, when (2S,3R,4S)-HIL is formulated as an oral preparation, an excipient and, where necessary, binder, disintegrant, lubricant, colorant, corrigent, flavor and the like are added, and produced as, for example, tablet, powder, pill, granule, capsule, suppository, solution, sugar coating agent, depot, syrup and the like by a conventional method. Examples of the excipient include lactose, cornstarch, sucrose, glucose, sorbit, crystalline cellulose and the like, examples of the binder include polyvinyl alcohol, polyvinyl ether, ethylcellulose, methylcellulose, gum arabic, tragacanth, gelatin, shellac, hydroxypropylcellulose, hydroxypropylstarch, polyvinylpyrrolidone and the like, examples of the disintegrant include starch, agar, gelatin powder, crystalline cellulose, calcium carbonate, sodium hydrogen carbonate, calcium citrate, dextran, pectin and the like, and examples of the lubricant include magnesium stearate, talc, polyethylene glycol, silica, hydrogenated vegetable oil and the like. As a colorant, those approved for addition to pharmaceutical products are used, and as a corrigent and a flavor, cocoa powder, menthol, aromatic acid, peppermint oil, borneol, powdered cinnamon bark and the like are used. These tablet and granule may be appropriately coated with sugar coating, gelatin coating or other necessary coating without any problem.
When an injection is prepared, where necessary, pH adjuster, buffer, stabilizer, preservative and the like are added and a subcutaneous, intramuscular or intravenous injection is produced by a conventional method.

As mentioned above, (2S,3R,4S)-HIL can be used as an agent for the prophylaxis or treatment of diabetes and can also be used in combination with other general therapeutic agent for diabetes or an agent for the prophylaxis or treatment of diabetic complications. The general therapeutic agent for diabetes and agent for the prophylaxis or treatment for diabetic complications include, for example, a combination or mixture of one or more kinds of insulin preparation, insulin derivative, drug having insulin-like activity, insulin secretagogue, insulin sensitizer, biguanide, gluconeogenesis inhibitor, sugar absorption inhibitor, kidney sugar reabsorption inhibitor, β3 adrenoceptor agonist, glucagon-like peptide-1(7-37), glucagon-like peptide-1(7-37) analogs, glucagon-like peptide-1 receptor agonist, dipeptidyl peptidase IV inhibitor, aldose reductase inhibitor, advanced glycation end product formation inhibitor, glycogen synthase kinase-3 inhibitor, glycogen phosphorylase inhibitor, antihyperlipidemia agent, appetite depressant, lipase inhibitor, antihypertensive agent, peripheral circulation improving drug, antioxidant, therapeutic drug for diabetic neuropathy and the like.

A pharmaceutical agent to be used in combination with (2S,3R,4S)-HIL may be mixed therewith to give a mixed preparation, or they may be individually formulated into preparations, or a combination preparation (set, kit, pack etc.) containing separately formulated preparations in one container may be used.

The administration mode of the combined use is not particularly limited, and, for example, (1) administration of a single preparation, (2) simultaneous administration of separate preparations by the same administration route, (3) administration of separate preparations by the same administration route in a staggered manner, (4) simultaneous administration of separate preparations by different administration routes, (5) administration of separate preparations by different administration routes in a staggered manner and the like can be mentioned.

Specific compounds of pharmaceutical agents to be used in combination and preferable diseases to be treated are exemplified in the following, which are not to be construed as limitative.

Examples of the insulin preparation include NPH, lente, ultralente, transpulmonarily absorbable insulin and the like.
The insulin derivative refers to protein or peptide retaining an insulin action induced from insulin and examples thereof include lispro insulin, B10Asp, glargine and the like.
The drug having an insulin-like activity is a drug other than insulin derivatives, which shows a hypoglycemic activity by exhibiting a physiological action of insulin such as a promoting action of sugar uptake into a cell and the like without depending on insulin to some extent, such as an insulin receptor kinase stimulating agent (e.g., L-783281, TER-17411, CLX-0901, KRX-613 etc.), vanadium and the like.

The insulin secretagogue refers to one that exhibits a hypoglycemic action by acting on pancreatic B cell to increase insulin secretion into the blood. Examples thereof include sulfonylurea (e.g., tolbutamide, chlorpropamide, tolazamide, acetohexamide, gliclazide, glimepiride, Glipizide, glibenclamide (glyburide) etc.), meglitinides (e.g., nateglinide, repaglinide, mitiglinide etc.), ATP sensitive potassium channel blocker (e.g., BTS-67-582 etc.) other than sulfonylureas and meglitimides, and the like.

The insulin sensitizer refers to one that exhibits a hypoglycemic activity by enhancing an insulin action in insulin target tissues. Examples thereof include peroxisome proliferator-activated receptor (PPAR) γ agonist (e.g., thiazolidinedione compounds such as pioglitazone, rosiglitazone, troglitazone, ciglitazone and the like, nonthiazolidinedione compounds such as GI-262570, GW-1929, JTT-501, YM-440 and the like, etc.), PPARγ antagonist (e.g., bisphenol A diglycidyl ether, LG-100641 etc.), PPARα agonist (fibrate compounds such as clofibrate, bezafibrate, clinofibrate and the like, nonfibrate compounds etc.), PPARα/γ agonist (e.g., KRP-297 etc.), retinoid X receptor agonist (e.g., LG-100268 etc.), retinoid X receptor antagonist (e.g., HX531 etc.), protein tyrosine phosphatase-1B inhibitor (e.g., PTP-112 etc.) and the like.

The biguanide refers to one that exhibits a hypoglycemic activity by a gluconeogenesis suppressive action in the liver, an anaerobic glycolysis promoting action in tissues, a peripheral insulin sensitizing activity and the like. Examples thereof include metformin, phenformin, buformin and the like.
The gluconeogenesis inhibitor refers to one that exhibits a hypoglycemic action mainly by inhibiting gluconeogenesis. Examples thereof include glucagon secretion suppressant (e.g., M&B 39890A etc.), glucagon receptor antagonist (e.g., CP-99711, NNC-92-1687, L-168049, BAY27-9955 etc.), glucose-6-phosphatase inhibitor and the like.

The sugar absorption inhibitor refers to one that exhibits a hypoglycemic action by inhibiting enzyme digestion of hydrocarbonate contained in diet in the gastrointestinal tract, and inhibiting or delaying intracorporeal absorption of sugar. Examples thereof include α-glucosidase inhibitor (e.g., acarbose, voglibose, miglitol etc.), α-amylase inhibitor (e.g., AZM-127 etc.) and the like.
The kidney sugar reabsorption inhibitor refers to one that exhibits a hypoglycemic action by inhibiting sugar reabsorption in the renal tubule. Examples thereof include sodium dependency glucose transporter inhibitor (e.g., T-1095, phloridzin etc.) and the like.

The β3 adrenoceptor agonist refers to one that exhibits an improving effect on obesity and hyperinsulinemia by stimulating β3 adrenoceptor which is present in adipocytes and promoting fatty acid oxidation to induce energy consumption. Examples thereof include CL-316243, TAK-677 and the like.
Examples of the glucagon-like peptide-1(7-37) analogs include exendin-4, NN-2211 and the like, examples of the glucagon-like peptide-1 receptor agonist include AZM-134 and the like, and examples of the dipeptidyl peptidase IV inhibitor include NVP-DPP-728 and the like. The glucagon-like peptide-1 analogs, glucagon-like peptide-1 receptor agonist, dipeptidyl peptidase IV inhibitor and glucagon-like peptide-1 refers to one that exhibits a diabetes-improving effect by mimicking or enhancing the action of cellular glucagon-like peptide-1.
The aldose reductase inhibitors refers to one that decreases, by inhibiting aldose reductase, intracellular sorbitol accumulated in excess from among those preferable for the treatment of diabetic complications. Such excess accumulation is due to the promotion of polyol metabolism pathway, which is caused by persistence of hyperglycemic state, which is observed in a tissue where diabetic complications are developed. Examples thereof include epalrestat, tolrestat, fidarestat, zenarestat and the like.

The advanced glycation end products formation inhibitor refers to one that relieves cellular dysfunction by inhibiting enhanced formation of advanced glycation end products due to persistent hyperglycemia state in a diabetes state, from among those preferable for the treatment of diabetic complications. The above-mentioned agent for the prophylaxis or treatment of diabetic complications is also included in this category. For combined use with the above-mentioned agent for the prophylaxis or treatment of diabetic complications, those other than the above-mentioned agents for the prophylaxis or treatment of diabetic complications can be used. Examples thereof include NNC-39-0028, OPB-9195 and the like.
Examples of the glycogen synthase kinase-3 inhibitor include SB-216763, CHIR-98014 and the like, and examples of the glycogen phosphorylase inhibitor include CP-91149 and the like.

Examples of the antihyperlipidemia agent include hydroxymethylglutaryl coenzyme A reductase inhibitor (e.g., pravastatin, simvastatin, fluvastatin, atorvastatin etc.), fibrate pharmaceutical agent (e.g., clofibrate, bezafibrate, simfibrate etc.), bile acid-binding resin and the like.
Examples of the appetite depressant include sibutramine, mazindol and the like, and examples of the lipase inhibitor include orlistat and the like.
Examples of the antihypertensive agent include angiotensin converting enzyme inhibitor (e.g., captopril, alacepril etc.), angiotensin II receptor antagonist (e.g., candesartan cilexetil, valsartan etc.), calcium antagonist (e.g., cilnidipine, amlodipine, nicardipine etc.), diuretic (e.g., trichlormethiazide, spironolactone etc.), sympatholytic agent (e.g., clonidine, reserpine etc.) and the like.
Examples of the peripheral circulation improvement drug include ethyl icosapentate and the like.
Examples of the antioxidant include lipoic acid, probucol and the like.
Examples of the therapeutic drug for diabetic neuropathy include mecobalamin, mexiletine hydrochloride and the like.

In addition, (2S,3R,4S)-HIL is also useful as a food additive.
A food composition containing (2S,3R,4S)-HIL is also useful as a food for the prophylaxis or treatment of diabetes.

While the "food" used in the present specification refers to foods in general, common foods, including what are called health foods, foods for specified health uses, and foods with nutrient function claims stipulated in the food with health claims system of the Ministry of Health, Labor and Welfare, can be mentioned, and dietary supplements can also be mentioned.

The form of the food composition described in the present specification is not particularly limited, and any form can be employed as long as it permits oral ingestion.
Examples thereof include powder, granule, tablet, hard capsule, soft capsule, liquid (drink, jellydrink etc.), candy, chocolate and the like, which can be produced by a method known per se in the art.
The content of the (2S,3R,4S)-HIL in a food composition can be appropriately determined such that a suitable dose in the indicated range can be obtained.

The above-mentioned food composition can contain other food additives as necessary. Examples of the food additive include fruit juice, dextrin, cyclic oligosaccharide, saccharides (monosaccharides such as fructose, glucose and the like and polysaccharides), acidulant, flavor, green tea powder, and the like, which control and improve the taste, emulsifier, collagen, whole milk powder, thickening polysaccharides, agar and the like, which improve texture, and further, those used as components of general health food and the like, such as vitamins, egg shell calcium, calcium pantothenate, other minerals, royal jelly, propolis, honey, dietary fiber, agaricus, chitin, chitosan, flavonoids, carotenoids, lutein, Kampo herbs, chondroitin, various amino acids and the like.

### Examples

The present invention is explained in more detail in the following by referring to Examples, which are not to be construed as limitative.

### (Experimental Example)

### [analysis conditions]

(1) ¹H-NMR: measured by Varian Mercury 300VX (300 M Hz)
(2) LCMS:
   (HPLC): Hewlett Packard series 1100
   column: Symmetry (registered trade mark) C18 3.5 µm 4.6×50 mm
   mobile phase: 0.05% TFA-containing water and 0.04% TFA-containing acetonitrile
   gradient: conditions under which 0.04% TFA-containing acetonitrile linearly increases from 5% to 98% in 5 min
   flow rate: 2 ml/min
   column temperature: 30°C
   detection: UV detector (210 nm - 300 nm)
   (Mass): ESI and PDA were detected using Micromass platform manufactured by Waters Corporation

### Example 1: purification of (2S,3R,4S)-HIL from seeds of Fenugreek

### (step 1) extraction of 4-hydroxyisoleucine crude product (HIL crude product)

The seeds of Fenugreek (5 g) were pulverized in a mill, defatted with hexane (50 ml), and then immerse in 70% aqueous ethanol solution (50 ml) overnight. The residue was collected by filtration, and washed with 70% ethanol (60 ml). The filtrate was evaporated under reduced pressure to give an HIL crude product.

### (step 2) aminalization with p-nitrobenzaldehyde and extraction with organic solvent

The HIL crude product obtained above was mixed with 5% aqueous sodium hydrogen carbonate solution (10 ml), a solution of p-nitrobenzaldehyde (0.2 g) in acetonitrile (3 ml) was added, and the mixture was stirred at 25°C overnight. Acetonitrile was evaporated under reduced pressure, and excess aldehyde was washed with ethyl acetate (Kanto Chemical Co., Inc., first grade, 99% or above) (50 ml). The obtained aqueous layer was neutralized with 1M aqueous citric acid solution, and the mixture was extracted with ethyl acetate. The ethyl acetate layer was washed with saturated brine, dried over anhydrous magnesium sulfate and the desiccant was filtered off to give a solution of an aminal form, (2R,4S,5R,6S)-5,6-dimethyl-2-(4-nitrophenyl)-tetrahydro-2H-1,3-oxazin-4-carboxylic acid, in ethyl acetate.
MS(ESI)m/z 281 [M+H]⁺

### (step 3) deaminalization

To the solution of (2R,4S,5R,6S)-5,6-dimethyl-2-(4-nitrophenyl)-tetrahydro-2H-1,3-oxazin-4-carboxylic acid in ethyl acetate obtained in the above-mentioned step was added 50% aqueous hydroxylamine solution (0.1 ml) and the mixture was stood for 3 days. The precipitated crystals were collected by filtration to give (2S,3R,4S)-4-hydroxyisoleucine (30 mg). This crystal showed one peak by LCMS and no impurity peak by ¹H-NMR, whereby the crystal was confirmed to be (2S,3R,4S)-HIL having a high purity.
¹H-NMR(300 MHz, D₂O) δ=0.83 (3H, d, J=7.2 Hz), 1.12 (3H, d, J=6.3 Hz), 1.75-1.83 (1H, m), 3.70 (1H, m), 3.76 (1H, d, J=7.8 Hz) MS(ESI)m/z 148 [M+H]⁺

### Example 2: aminalization of stereoisomer mixture of 4-hydroxyisoleucine with p-nitrobenzaldehyde (synthesis of (2R,4S,5R,6S)-5,6-dimethyl-2-(4-nitrophenyl)-tetrahydro-2H-1,3-oxazin-4-carboxylic acid)

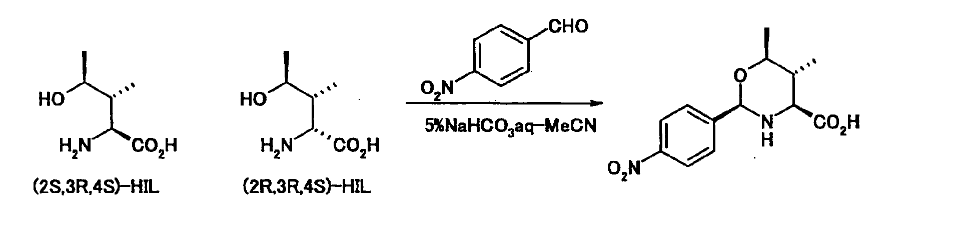

An about 1:1 mixture (0.4 g) of (2S,3R,4S)-4-hydroxyisoleucine and (2R,3R,4S)-4-hydroxyisoleucine was mixed with 5% aqueous sodium hydrogen carbonate solution (10 ml), a solution of p-nitrobenzaldehyde (0.41 g) in acetonitrile (20 ml) was added and the mixture was stirred overnight. Acetonitrile was evaporated under reduced pressure, and excess aldehyde was washed with ethyl acetate. The obtained aqueous layer was neutralized with 1M hydrochloric acid solution, and the mixture was extracted with ethyl acetate. The ethyl acetate layer was washed with saturated brine, and dried over anhydrous magnesium sulfate. The desiccant was filtered off and the solvent was rapidly evaporated under reduced pressure to give (2R,4S,5R,6S)-5,6-dimethyl-2-(4-nitrophenyl)-tetrahydro-2H-1,3-oxazin-4-carboxylic acid (stereoisomer corresponding to (2S,3R,4S)-HIL) (0.3 g). The obtained solid showed one peak by LCMS and did not show no peak derived from stereoisomer corresponding to (2R,3R,4S)-HIL by ¹H-NMR. In addition, the absolute configuration of the obtained compound was also supported by NOESY spectrum thereof. ¹H-NMR(300 MHz, CD₃OD) δ =1.00(3H, d, J=6.6 Hz), 1.34(3H, d, J=6.0 Hz), 1.45-1.58(1H, m), 3.47(1H, d, J=10.5 Hz), 3.60-3.70(1H, m), 5.39(1H, s), 7.76(2H, d, J=8.7 Hz), 8.23(2H, d, J=8.7 Hz)
MS(ESI)m/z 281 [M+H]⁺

NOESY spectrum was measured and the following cross-peak was observed.

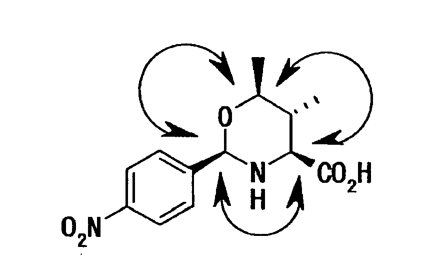

That is, the obtained compound was confirmed to be (2R,4S,5R,6S)-5,6-dimethyl-2-(4-nitrophenyl)-tetrahydro-2H-1,3-oxazin-4-carboxylic acid having the following stereo configuration.

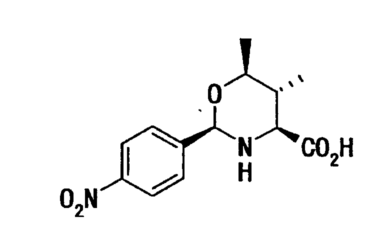

### Example 3: deaminalization reaction with hydroxylamine

32

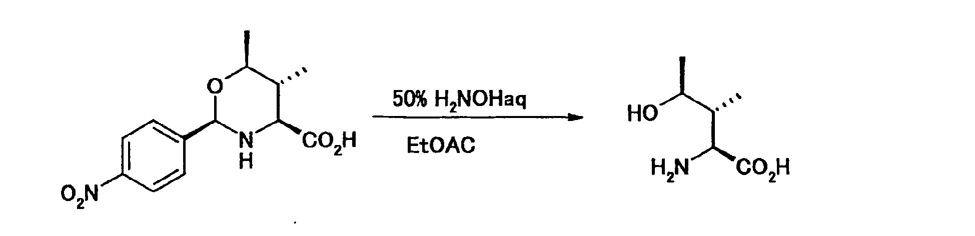

(2R,4S,5R,6S)-5,6-Dimethyl-2-(4-nitrophenyl)-tetrahydro-2H-1,3-oxazin-4-carboxylic acid (50 mg, 0.18 mmol) obtained by an operation similar to that in Example 2 was dissolved in ethyl acetate (2 ml), 50% aqueous hydroxylamine solution (0.01 ml) was added, and the mixture was stirred for 2 hr. The precipitated crystals were collected by filtration to give (2S,3R,4S)-4-hydroxyisoleucine (7 mg). The obtained crystals showed one peak by LCMS and no impurity peak by ¹H-NMR, whereby the crystal was confirmed to be (2S,3R,4S)-4-hydroxyisoleucine having a high purity.
¹H-NMR(300 MHz, D₂O)δ=0.83(3H, d, J=7.2 Hz), 1.12(3H, d, J=6.3 Hz), 1.75-1.83(1H, m), 3.70(1H, m), 3.76(1H, d, J=7.8 Hz) MS(ESI)m/z 148 [M+H]⁺

### Example 4: deaminalization reaction with methylamine methanol solution

By an operation according to Example 3 and using 40% methylamine methanol solution (0.014 ml) instead of the 50% aqueous hydroxylamine solution, (2S,3R,4S)-HIL (4 mg) could be obtained. The obtained crystals showed one peak by LCMS and no impurity peak by ¹H-NMR, whereby the crystals were confirmed to be (2S,3R,4S)-4-hydroxyisoleucine having a high purity.

### Example 5: deaminalization reaction with hydrazine monohydrate

By an operation according to Example 3 and using hydrazine monohydrate (0.01 ml) instead of the 50% aqueous hydroxylamine solution, (2S,3R,4S)-HIL (20 mg) could be obtained. The obtained crystals showed one peak by LCMS and no impurity peak by ¹H-NMR, whereby the crystals were confirmed to be (2S,3R,4S)-4-hydroxyisoleucine having a high purity.

### Example 6: aminalization of stereoisomer mixture of 4-hydroxyisoleucine with cyclohexyl aldehyde (synthesis of (2R,4S,5R,6S)-2-cyclohexyl-5,6-dimethyl-tetrahydro-2H-1,3-oxazin-4-carboxylic acid)

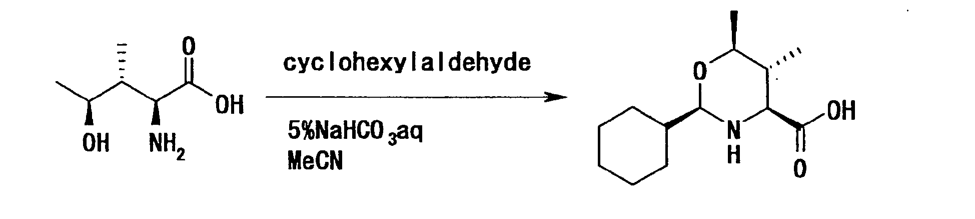

An about 3:2 mixture (0.1 g, 0.68 mmol) of (2S,3R,4S)-HIL and (2R,3R,4S)-HIL was dissolved in 5% aqueous sodium hydrogen carbonate solution (2.3 ml), a solution of cyclohexyl aldehyde (0.076 g, 0.68 mmol) in acetonitrile (2.3 ml) was added and the mixture was stirred overnight. Acetonitrile was evaporated under reduced pressure, and excess aldehyde was washed with ethyl acetate. The obtained aqueous layer was neutralized with 2M aqueous citric acid solution, and the mixture was extracted with ethyl acetate. The ethyl acetate layer was washed with saturated brine, and dried over anhydrous magnesium sulfate. The desiccant was filtered off and the solvent was rapidly evaporated under reduced pressure to give (2R,4S,5R,6S)-2-cyclohexyl-5,6-dimethyl-tetrahydro-2H-1,3-oxazin-4-carboxylic acid (0.067 g).
¹H-NMR(300 MHz, D₂O)δ=1.06(3H, d, J=6.6 Hz), 1.07-1.40(6H, m), 1.60-2.00(9H, m), 3.34(1H, d, J=11.4 Hz), 3.42-3.50(1H, m), 4.24(1H, d, J=6.6 Hz)
MS(ESI)m/z 242 [M+H]⁺

### Example 7: deaminalization reaction of (2R,4S,5R,6S)-2-cyclohexyl-5,6-dimethyl-tetrahydro-2H-1,3-oxazin-4-carboxylic acid with hydroxylamine

(2R,4S,5R,6S)-2-Cyclohexyl-5,6-dimethyl-tetrahydro-2H-1,3-oxazin-4-carboxylic acid (0.067 g, 0.42 mmol) obtained in Example 6 was dissolved in methanol (3 ml), 50% aqueous hydroxylamine solution (0.017 ml) was added, and the mixture was stirred for 2 hr. The solvent was evaporated under reduced pressure, ethyl acetate was added to the obtained residue, and insoluble crystals were collected by filtration to give (2S,3R,4S)-4-hydroxyisoleucine (28 mg). The obtained crystals showed one peak by LCMS and no impurity peak by ¹H-NMR, whereby the crystal was confirmed to be (2S,3R,4S)-4-hydroxyisoleucine having a high purity.

### Example 8: aminalization of stereoisomer mixture of HIL with benzaldehyde (synthesis of (2R,4S,5R,6S)-5,6-dimethyl-2-phenyl-tetrahydro-2H-1,3-oxazin-4-carboxylic acid)

### (step 1) aminalization with benzaldehyde and extraction with organic solvent

An about 1:1 mixture (0.1 g) of (2S,3R,4S)-HIL and (2R,3R,4S)-HIL was dissolved in 5% aqueous sodium hydrogen carbonate solution (2.7 ml), a solution of benzaldehyde (0.084 g) in acetonitrile (2.7 ml) was added, and the mixture was stirred overnight. Acetonitrile was evaporated under reduced pressure, and excess aldehyde was washed with ethyl acetate (Kanto Chemical Co., Inc., first grade, 99% or above). The obtained aqueous layer was neutralized with 1M aqueous citric acid solution, and the mixture was extracted with ethyl acetate. The ethyl acetate layer was washed with saturated brine, dried over anhydrous magnesium sulfate and the desiccant was filtered off to give a solution of (2R,4S,5R,6S)-5,6-dimethyl-2-phenyl-tetrahydro-2H-1,3-oxazin-4-carboxylic acid in ethyl acetate.

### (step 2) deaminalization reaction by catalytic reduction

The ethyl acetate layer was stood in the air at room temperature to give (2S,3R,4S)-HIL (12 mg) as precipitated crystals having a high purity (¹H-NMR and LCMS). The crystals were removed by filtration and an aminal form in the filtrate was converted to (2S,3R,4S)-HIL as follows.
The solvent in the filtrate was evaporated under reduced pressure, and a 1:2 mixed solvent of water and methanol and a catalytic amount of 10% palladium carbon were added to the residue, and the mixture was stirred overnight after purging the system with hydrogen. The palladium carbon was filtered off, and (2S,3R,4S)-HIL (40 mg) was obtained by concentration under reduced pressure and freeze-drying. The freeze-dried product showed one peak by LCMS.

### Example 9: aminalization with various aldehydes

The following compounds were obtained in the same manner as in Example 6.

### (2R,4S,5R,6S)-5,6-dimethyl-2-phenyl-tetrahydro-2H-1,3-oxazin-4-carboxylic acid

¹H-NMR(300 MHz, CD₃OD)δ=1.09(3H, d, J=6.6 Hz), 1.35(3H, d, J=6.3 Hz), 1.63-1.78(1H, m), 3.53(1H, d, J=10.8 Hz), 3.68-3.74(1H, m), 5.47(1H, s), 7.40-7.44(3H, m), 7.54-7.60(2H, m) MS(ESI)m/z 236 [M+H]⁺

### (2R,4S,5R,6S)-5,6-dimethyl-2-(4-pyridyl)-tetrahydro-2H-1,3-oxazin-4-carboxylic acid

¹H-NMR(300 MHz, CD₃OD)δ=0.98(3H, d, J=6.6 Hz), 1.34(3H, d, J=6.3 Hz), 1.42-1.58(1H, m), 3.45(1H, d, J=10.8 Hz), 3.60-3.68(1H, m), 5.31(1H, s), 7.59(2H, d, J=4.8 Hz), 8.54(2H, d, J=4.8 Hz)
MS(ESI)m/z 237 [M+H]⁺

### (2R,4S,5R,6S)-2-(4-chlorophenyl)-5,6-dimethyl-tetrahydro-2H-1,3-oxazin-4-carboxylic acid

MS(ESI)m/z 270 [M+H]⁺

### (2R,4S,5R,6S)-5,6-dimethyl-2-(4-methoxyphenyl)-tetrahydro-2H-1,3-oxazin-4-carboxylic acid

MS(ESI)m/z 266 [M+H]⁺

### (2R,4S,5R,6S)-5,6-dimethyl-2-(4-methylphenyl)-tetrahydro-2H-1,3-oxazin-4-carboxylic acid

MS(ESI)m/z 250 [M+H]⁺

### (2R,4S,5R,6S)-5,6-dimethyl-2-(4-trifluoromethylphenyl)-tetrahydro-2H-1,3-oxazin-4-carboxylic acid

MS(ESI)m/z 304 [M+H]⁺

### (2R,4S,5R,6S)-5,6-dimethyl-2-(4-fluorophenyl)-tetrahydro-2H-1,3-oxazin-4-carboxylic acid

MS(ESI)m/z 254 [M+H]⁺

### (2R,4S,5R,6S)-2-(4-cyanophenyl)-5,6-dimethyl-tetrahydro-2H-1,3-oxazin-4-carboxylic acid

MS(ESI)m/z 261 [M+H]⁺

### (2R,4S,5R,6S)-2-(2-chlorophenyl)-5,6-dimethyl-tetrahydro-2H-1,3-oxazin-4-carboxylic acid

MS(ESI)m/z 270 [M+H]⁺

### (2R,4S,5R,6S)-5,6-dimethyl-2-(2-nitrophenyl)-tetrahydro-2H-1,3-oxazin-4-carboxylic acid

MS(ESI)m/z 281 [M+H]⁺

### (2R,4S,5R,6S)-5,6-dimethyl-2-(3-nitrophenyl)-tetrahydro-2H-1,3-oxazin-4-carboxylic acid

MS(ESI)m/z 281 [M+H]⁺

### (2R,4S,5R,6S)-5,6-dimethyl-2-(2-thienyl)-tetrahydro-2H-1,3-oxazin-4-carboxylic acid

MS(ESI)m/z 242 [M+H]⁺

### (2R,4S,5R,6S)-5,6-dimethyl-2-(2-furyl)-tetrahydro-2H-1,3-oxazin-4-carboxylic acid

MS(ESI)m/z 226 [M+H]⁺

### Industrial Applicability

According to the method of the present invention, (2S,3R,4S)-4-HIL having a high purity can be obtained efficiently in a high yield. The obtained (2S,3R,4S)-4-HIL is useful as, for example, a pharmaceutical product (therapeutic agent for diabetes etc.), a health food material, a cosmetic material and the like, and also useful as a synthetic intermediate for various pharmaceutical products.
In addition, the method of the present invention is useful as a method of separating and removing (2S,3R,4S)-HIL, and can efficiently remove (2S,3R,4S)-HIL from a mixture of compounds.
This application is based on patent application Nos. 2007-042711 and 2007-144578 filed in Japan, the contents of which are incorporated in full herein by this reference. In addition, the patent documents and non-patent documents cited in the present specification are hereby incorporated in their entireties by reference, to the extent that they have been disclosed in the present specification.

## Claims

1. A method of purifying (2S,3R,4S)-4-hydroxyisoleucine or a chemically acceptable salt thereof, which comprises the following steps (a), (b) and (c):
(a) a step of reacting (2S,3R,4S)-4-hydroxyisoleucine or a chemically acceptable salt thereof in a mixture with an aldehyde compound represented by the formula: Q-CHO wherein Q is an aryl group having a carbon number of 6 to 14, an alkyl group having a carbon number of 1 to 10, a cycloalkyl group having a carbon number of 3 to 10 or a 5- to 10-membered heterocyclic group, each of which optionally has substituent(s), or an equivalent form thereof to give a compound represented by the formula (1) 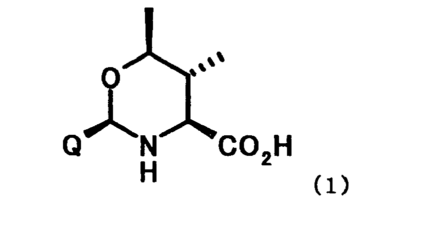 wherein Q is as defined above,
(b) a step of extracting the compound represented by the formula (1) with an organic solvent, and
(c) a step of converting the compound represented by the formula (1) to (2S,3R,4S)-4-hydroxyisoleucine or a chemically acceptable salt thereof.

2. The method of claim 1, wherein an aldehyde compound represented by the formula: Q-CHO is used for the reaction of step (a).

3. The method of claim 1, comprising removing impurities from the compound represented by the formula (1) by the extraction step of step (b).

4. The method of claim 1 or 2, wherein Q is a phenyl group, a cyclohexyl group, a naphthyl group, a pyridyl group, a thienyl group, a furyl group or a pyrrolyl group, each of which optionally has substituent(s).

5. The method of claim 4, wherein Q in the formula (1) is a phenyl group optionally having substituent(s).

6. The method of claim 4 or 5, wherein the substituent optionally possessed by Q is selected from the group consisting of
(i) an alkyl group having a carbon number of 1 to 6,
(ii) an alkoxy group having a carbon number of 1 to 6,
(iii) an alkanoyl group having a carbon number of 2 to 7,
(iv) an aryl group having a carbon number of 6 to 10,
(v) an aryloxy group having a carbon number of 6 to 10,
(vi) a halogeno group,
(vii) a halogenoalkyl group having a carbon number of 1 to 6,
(viii) a nitro group,
(ix) a formyl group, and
(x) a cyano group,
provided that the above-mentioned (i) and (vii) are not substituents of an alkyl group having a carbon number of 1 to 10.

7. The method of claim 1, wherein the organic solvent of step (b) is at least one kind of alkyl acetate.

8. The method of claim 1, which uses, in step (c), at least one kind of nucleophilic reagent selected from the group consisting of water, hydroxylamine optionally substituted by alkyl having a carbon number of 1 to 4, ammonia, alkylamine having a carbon number of 1 to 4, dialkylamine having a carbon number of 2 to 8, hydrazine optionally substituted by substituent(s) selected from an alkyl group having a carbon number of 1 to 4, an aryl group having a carbon number of 6 to 10 and an alkanoyl group having a carbon number of 2 to 7, quaternary ammonium halide substituted by alkyl having a carbon number of 1 to 6, and a chemically acceptable salt thereof.

9. The method of claim 1, wherein the conversion to (2S,3R,4S)-4-hydroxyisoleucine or a chemically acceptable salt thereof is performed in at least one kind of alkyl acetate in step (c).

10. The method of claim 9, wherein the at least one kind of alkyl acetate is ethyl acetate.

11. A method of separating and removing (2S,3R,4S)-4-hydroxyisoleucine or a chemically acceptable salt thereof, comprising the following steps (a) and (b):
(a) a step of reacting (2S,3R,4S)-4-hydroxyisoleucine or a chemically acceptable salt thereof in a mixture with an aldehyde compound represented by the formula: Q-CHO wherein Q is an aryl group having a carbon number of 6 to 14, an alkyl group having a carbon number of 1 to 10, a cycloalkyl group having a carbon number of 3 to 10 or a 5- to 10-membered heterocyclic group, each of which optionally has substituent(s), or an equivalent form thereof to give a compound represented by the formula (1) 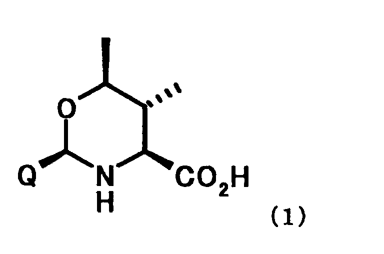 wherein Q is as defined above, and
(b) a step of extracting the compound represented by the formula (1) with an organic solvent.

12. The method of claim 11, wherein an aldehyde compound represented by the formula: Q-CHO is used for the reaction in step (a).

13. The method of claim 11 or 12, wherein Q in the formula (1) is a phenyl group, a cyclohexyl group, a naphthyl group, a pyridyl group, a thienyl group, a furyl group or a pyrrolyl group, each of which optionally has substituent(s).

14. The method of claim 13, wherein Q in the formula (1) is a phenyl group optionally having substituent(s).

15. The method of claim 13 or 14, wherein the substituent optionally possessed by Q is selected from the group consisting of
(i) an alkyl group having a carbon number of 1 to 6,
(ii) an alkoxy group having a carbon number of 1 to 6,
(iii) an alkanoyl group having a carbon number of 2 to 7,
(iv) an aryl group having a carbon number of 6 to 10,
(v) an aryloxy group having a carbon number of 6 to 10,
(vi) a halogeno group,
(vii) a halogenoalkyl group having a carbon number of 1 to 6,
(viii) a nitro group,
(ix) a formyl group, and
(x) a cyano group,
provided that the above-mentioned (i) and (vii) are not used as substituents of an alkyl group having a carbon number of 1 to 10.

16. The method of claim 11, wherein the organic solvent in step (b) is at least one kind of alkyl acetate.
